# FASCICULE DE BREVET EUROPEEN

(11) **EP 3 655 529 B1**
(45) Date de publication et mention de la délivrance du brevet: **25.06.2025**
(21) Numéro de dépôt: 18756269.9
(22) Date de dépôt: 17.07.2018
(51) Int. Cl.: C12N 9/16, A61K 38/46

(54) **NOUVELLES ENZYMES PTE MUTÉES**
NEUE MUTIERTE PTE-ENZYME
NEW MUTATED PTE ENZYMES

(30) Priorité: 17.07.2017 FR 1756776
(43) Date de publication de la demande: 27.05.2020
(73) Titulaire: La Fondation Mediterranée Infection, 13005 Marseille (FR); Gene And Green TK, 13005 Marseille (FR); Assistance Publique - Hôpitaux de Marseille, 13005 Marseille (FR); Centre National de la Recherche Scientifique, 75794 Paris Cedex 16 (FR); Aix-Marseille Université, 13331 Marseille Cedex 3 (FR); Regents of the University of Minnesota, Minneapolis, MN 55455 (US)
(72) Inventeur: CHABRIERE, Eric, 13008 Marseille (FR); DAUDE, David, 13005 Marseille (FR); ELIAS, Mikael, 57190 Florange (FR)
(74) Mandataire: Grosset-Fournier, Chantal Catherine
(86) Numéro de dépôt international: PCT/FR2018/051822
(87) Numéro de publication internationale: WO 2019/016468

(56) Documents cités:
- WO-A1-2013/010225
- WO-A1-2015/196106
- WO-A2-2016/092555

## Description

La présente demande décrit des enzymes phosphotriesterases mutées possédant une stabilité et une activité améliorée, ainsi que leur utilisation notamment pour dégrader des composés organophosphorés.

Les insecticides organophosphorés (OP) sont devenus les insecticides les plus utilisés de nos jours. Les OP sont utilisés dans l'agriculture, au sein du domicile, dans le cadre du jardinage ainsi que dans le secteur vétérinaire.

Comme ces composés inhibent certaines enzymes estérases, une exposition aux OP peut mener à une grave toxicité, de plusieurs façons.

Une inhibition irréversible de l'acétylcholinestérase (une enzyme clef du système nerveux chez les mammifères) par les OP engendre de graves dommages chez tous les vertébrés. Une perte de fonction de l'enzyme mène à l'accumulation de l'acétylcholine dans différents compartiments du corps, engendrant une contraction musculaire, une paralysie et une détresse respiratoire. L'augmentation des sécrétions pulmonaires combinée à une défaillance du système respiratoire constitue la cause habituelle des décès liés à un empoisonnement aux organophosphorés.

Certains OP ont aussi été développés durant la Seconde Guerre Mondiale : la découverte d'OP avec une toxicité augmentée et/ou une meilleure stabilité a mené au développement d'armes chimiques telles que le gaz sarin, soman, tabun ou VX. De plus, les insecticides OP étant facilement accessibles, et non moins toxiques comparés aux OP des armes chimiques, constituent un risque important pour la population.

Plus proche de nous, le risque terroriste actuel a mené les autorités à envisager des scénarii d'attaques chimiques à l'aide de substances organophosphorées telles que le gaz sarin et face à ces menaces grandissantes, le développement d'antidotes n'a jamais été aussi urgent.

Les OP sont facilement absorbés par l'organisme par inhalation, ingestion ou encore par pénétration cutanée à cause du caractère hydrophobe de ces molécules. L'incidence de l'empoisonnement dépend du taux d'absorption du composé. Les symptômes d'un empoisonnement aigüe aux OP se manifestent pendant ou après l'exposition, durant les minutes ou les heures qui suivent, suivant le type d'exposition.

L'exposition par inhalation entraine la manifestation la plus rapide des symptômes de l'intoxication, suivie par la voie gastro-intestinale et enfin, l'intoxication par contact cutané.

Les vêtements de protection et les masques n'offrent pas toujours une protection suffisante contre les OP. Chez les patients empoisonnés par les OP via la peau, les vêtements ou les cheveux, la décontamination doit s'effectuer avec un savon médical ou des détergents textiles. Le traitement des personnes hautement contaminées s'effectue en administrant de l'atropine ou du diazepam, qui antagonisent les effets d'une concentration excessive en acétylcholine aux organes cibles possédant des récepteurs muscariniques.

Le pralidoxime, un réactivateur de l'acétylcholinestérase, atténue les effets nicotiniques comme les effets muscariniques d'un empoisonnement aux OP quand il est administré moins de 48h après l'empoisonnement.

Bien que des progrès en termes de prophylaxie aient été faits avec les 10 techniques mentionnées précédemment, les protections et les traitements existants pour ces types d'empoisonnement demeurent insatisfaisants.

Les premières OP-hydrolases ont été identifiées dans plusieurs bactéries au début des années 90 (Cheng et al., 1993, Appl. Environ. Microbiol., 59: 3138-3140, Raveh et al., 1993, Biochem Pharmacol., 45: 2465-2474). Ces enzymes sont capables de catalyser l'hydrolyse des liaisons phosphotriester dans les OP. Malheureusement, à cause de leur faible capacité de liaison stœchiométrique aux OP, une grande quantité d'enzymes est nécessaire pour guérir les individus empoisonnés. Ceci rend l'usage de ces enzymes disproportionné et onéreux.

D'autres enzymes microbiennes généralement appelées phosphotriestérases (PTE) montrent une préférence pour les composés organophosphorés avec des liaisons P-O ou P-S. Ces enzymes sont membres d'une superfamille, les aminohydrolases qui sont des enzymes qui catalysent l'hydrolyse d'un large éventail de composés avec différentes propriétés chimiques (phosphoesters, esters, amides, etc.). Leurs gènes codants, *opd* (organophosphate degradation), ont été isolés dans des bactéries du sol telles que *Pseudomonas diminuta,* aussi appelée *Brevundimonas diminuta* (Munnecke et al., 1976, Appl. Environ. Microbiol., 32: 7-13), *Flavobacterium sp.* (Sethunathan et al., 1973, Can J Microbiol, 19: 873-875) et *Agrobacterium radiobacter* (Horne et al., 2003, FEMS Microbiol Lett, 222: 1-8), et des gènes similaires à opd ont aussi été identifiés dans les archées (Merone et al., 2005, Extremophiles, 9: 297-305).Les propriétés catalytiques des PTE hyperthermophiles sont minutieusement étudiées à cause de leur capacité à hydrolyser les pesticides et divers agents innervants (Jackson et al., 2005, Biochem Biophys Acta, 1752: 56-64/ Jackson et al., 2008, J Mol Biol, 375: 1189-1196/ Wong et al., 2007, Biochemistry, 46: 13352-13369/ Elias et al., 2008, J Mol Biol, 379: 1017-1028/ Pompea et al., 2009, Extremophiles, 13: 461-470).

Les PTE hyperthermophiles ont l'avantage d'être très stables et peu couteuses à produire grâce à leur capacité à résister aux solvants organiques ou aux détergents, à température modérée. Ainsi, les PTE hyperthermophiles sont prometteuses pour le développement de bioépurateurs pour des agents neurotoxiques.

L'invention est exposée dans le jeu de revendications joint.

L'un des buts de la présente demande est de décrire des enzymes PTE possédant une stabilité améliorée.

Un autre but de la présente demande est de décrire l'amélioration, en plus de la stabilité, de l'activité enzymatique d'une telle enzyme PTE.

Dans son aspect le plus général, la présente demande décrit une enzyme PTE mutée dérivée de la parathion hydrolase de séquence SEQ ID NO : 1, laquelle enzyme PTE mutée présente au moins 90% d'identité avec la SEQ ID NO : 1 et comprend au moins les 7 mutations suivantes par rapport à la séquence SEQ ID NO : 1 :
- Substitution de la thréonine T par la proline P, en position 13,
- Substitution de l'isoleucine I par la valine V, en position 14,
- Substitution de l'alanine A par la sérine S, en position 60,
- Substitution de la sérine S par l'arginine R, en position 79,
- Substitution de la tyrosine Y par l'histidine H, en position 124,
- Substitution de l'isoleucine I par la valine V, en position 218,
- Substitution de la glutamine Q par l'arginine R, en position 258.

Les inventeurs ont décrit que des mutations spécifiques dans la séquence de la parathion hydrolase de séquence SEQ ID NO : 1 permettent l'obtention d'une enzyme ayant conservé une activité catalytique substantiellement identique à celle de la parathion hydrolase sauvage, mais ayant une stabilité améliorée. La parathion hydrolase de séquence dite « sauvage » est l'enzyme de séquence SEQ ID NO : 1.

Par « au moins 90% d'identité », on désigne les plages de valeurs d'au moins 91%, au moins 92%, d'au moins 93%, d'au moins 94%, d'au moins 95%, d'au moins 96%, d'au moins 97%, d'au moins 98%, d'au moins 99% et de 100% d'identité.

Par « enzyme ayant une stabilité améliorée », on désigne une enzyme dont la température de fusion Tₘ est augmentée par rapport à celle de la parathion hydrolase non mutée.

Pour fixer les idées, l'augmentation de la stabilité peut se traduire par une augmentation de la température de fusion d'au moins 3% et notamment d'au moins 3% à 20%.

L'expression « activité catalytique substantiellement identique » correspond à une variation du rapport K_{cat}/Kₘ de l'enzyme mutée inférieure à un facteur 10 par rapport au ratio K_{cat}/Kₘ de l'enzyme non mutée.

A titre d'exemple, des rapports K_{cat}/Kₘ de 6.10⁵ M⁻¹.s⁻¹ et 3.10⁵ M⁻¹.s⁻¹ sont jugés suffisamment proches (facteur 2) pour considérer que les enzymes qui possèdent de tels rapports K_{cat}/Kₘ ont une activité catalytique substantiellement identique.

Par « position », on désigne la place d'un acide aminé dans la séquence polypeptidique, établie à partir de l'extrémité N-terminale de l'enzyme de séquence SEQ ID NO : 1.

L'ensemble des 7 mutations citées précédemment, aux positions 13, 14, 60, 79, 124, 218 et 258 par rapport à la séquence SEQ ID NO : 1 seront désignées par la suite comme constituant les mutations du groupe A.

Ainsi, l'expression « mutations du groupe A » pourra être replacée par la liste suivant :
- « Substitution de la thréonine T par la proline P, en position 13,
- Substitution de l'isoleucine I par la valine V, en position 14,
- Substitution de l'alanine A par la sérine S, en position 60,
- Substitution de la sérine S par l'arginine R, en position 79,
- Substitution de la tyrosine Y par l'histidine H, en position 124,
- Substitution de l'isoleucine I par la valine V, en position 218,
- Substitution de la glutamine Q par l'arginine R, en position 258. »

Par « pourcentage d'identité » par rapport à une séquence donnée, on désigne le pourcentage des acides aminés identiques à ceux d'une séquence de référence et qui se retrouvent aux mêmes positions. Un tel pourcentage d'identité est établi par un alignement bio-informatique de type BlastP.

Par « mutation », on considère une mutation ponctuelle, à savoir la présence d'un acide aminé à une position donnée qui est différent de celui d'une séquence de référence, la séquence SEQ ID NO : 1 étant choisie comme étant la séquence de référence.

Par substitution, on désigne le remplacement d'un acide aminé d'une séquence donnée par un acide aminé différent.

Dans un mode de réalisation particulier, la présente demande décrit une enzyme PTE mutée de séquence SEQ ID NO : 2, dérivée de la parathion hydrolase ayant la séquence SEQ ID NO : 1, laquelle enzyme PTE mutée présente au moins 90% d'identité avec la SEQ ID NO : 1 et comprend au moins les 7 mutations suivantes par rapport à la séquence SEQ ID NO : 1 :
- Substitution de la thréonine T par la proline P, en position 13,
- Substitution de l'isoleucine I par la valine V, en position 14,
- Substitution de l'alanine A par la sérine S, en position 60,
- Substitution de la sérine S par l'arginine R, en position 79,
- Substitution de la tyrosine Y par l'histidine H, en position 124,
- Substitution de l'isoleucine I par la valine V, en position 218,
- Substitution de la glutamine Q par l'arginine R, en position 258,
et enzyme mutée présentant un pourcentage d'identité d'au moins 90 % avec la susdite séquence SEQ ID NO : 2, sous réserve de la présence des 7 susdites mutations dans ladite séquence de l'enzyme mutée.

Selon un autre mode de réalisation particulier, la demande décrit une enzyme PTE mutée, comprenant le groupe A de mutations tel que défini ci-dessus, dans laquelle au moins 2 acides aminés supplémentaires choisis parmi les acides aminés occupant les positions suivantes sont mutés :
- Acide aminé en position 45,
- Acide aminé en position 48,
- Acide aminé en position 74,
- Acide aminé en position 100,
- Acide aminé en position 141,
- Acide aminé en position 153,
- Acide aminé en position 177,
- Acide aminé en position 201,
- Acide aminé en position 222,
- Acide aminé en position 225,
- Acide aminé en position 235,
- Acide aminé en position 238,
- Acide aminé en position 239,
- Acide aminé en position 240,
- Acide aminé en position 242,
- Acide aminé en position 271,
- Acide aminé en position 276,
- Acide aminé en position 277,
- Acide aminé en position 287,
- Acide aminé en position 310,
les positions étant définies par rapport à la séquence SED ID NO : 1.

Une telle enzyme peut être obtenue à partir de la séquence SEQ ID NO : 1 qui, en plus des mutations du groupe A, comprend 2 mutations supplémentaires aux positions définies ci-dessus. A titre d'exemple, on pourra, en plus des 7 mutations du groupe A évoquées, effectuer 2 mutations : l'une en position 141 et l'autre en position 277.

Par « mutation supplémentaire » ou « substitution supplémentaire », on désigne toute mutation ou substitution survenant en plus de celles du groupe A.

Selon un autre mode de réalisation particulier, la demande décrit une enzyme PTE mutée, comprenant les mutations du groupe A, dans laquelle les mutations supplémentaires aux positions définies ci-dessus sont choisies parmi la liste suivante :
- En position 45 : substitution de la lysine K par l'alanine A,
- En position 48 : substitution de l'alanine A par la valine V ou la méthionine M,
- En position 74 : substitution de l'isoleucine I par la cystéine C ou l'alanine A,
- En position 100 : substitution de la phénylalanine F par l'acide glutamique E ou la valine V,
- En position 141 : substitution de la thréonine T par l'asparagine N,
- En position 153 : substitution de la lysine K par l'arginine R,
- En position 177 : substitution de la glycine G par l'acide aspartique D,
- En position 201 : substitution de l'acide aspartique D par la glycine G,
- En position 222 : substitution de l'histidine H par la glycine G ou la glutamine Q,
- En position 225 : substitution de l'histidine H par la tyrosine Y,
- En position 235 : substitution de la sérine S par la méthionine M,
- En position 238 : substitution de la l'alanine A par la valine V ou la sérine S,
- En position 239 : substitution de la leucine L par le tryptophane W,
- En position 240 : substitution de la leucine L par la méthionine M,
- En position 242 : substitution de l'isoleucine I par l'asparagine N,
- En position 271 : substitution de la leucine L par la thréonine T,
- En position 276 : substitution de la sérine S par la leucine L,
- En position 277 : substitution de la tyrosine Y par le tryptophane W,
- En position 287 : substitution de l'arginine R par la sérine S,
- En position 310 : substitution de la proline P par la sérine S.

A titre d'exemple, une telle enzyme mutée peut être obtenue à partir de l'enzyme de séquence SEQ ID NO: 1 qui, en plus des 7 mutations du groupe A, comprend 2 supplémentaires, comme par exemple une substitution de l'alanine A en position 48 par la valine V ou la méthionine M et la substitution de la proline P en position 310 par la sérine S. Une telle enzyme mutée comprend alors 9 mutations par rapport à l'enzyme de séquence SEQ ID NO : 1, aux positions 13, 14, 48, 60, 79, 124, 218, 258 et 310.

Selon un autre mode de réalisation particulier, la demande décrit une enzyme PTE mutée comprenant uniquement le groupe A de mutations, de séquence SEQ ID NO : 2, dans laquelle :
- L'acide aminé en position 45 est la lysine K,
- L'acide aminé en position 48 est l'alanine A,
- L'acide aminé en position 74 est l'isoleucine I,
- L'acide aminé en position 100 est la phénylalanine F,
- L'acide aminé en position 141 est la thréonine T,
- L'acide aminé en position 153 est la lysine K,
- L'acide aminé en position 177 est la glycine G,
- L'acide aminé en position 201 est l'acide aspartique D,
- L'acide aminé en position 222 est l'histidine H,
- L'acide aminé en position 225 est l'histidine H,
- L'acide aminé en position 235 est la sérine S,
- L'acide aminé en position 238 est l'alanine A,
- L'acide aminé en position 239 est la leucine L,
- L'acide aminé en position 240 est la leucine L,
- L'acide aminé en position 242 est l'isoleucine I,
- L'acide aminé en position 271 est la leucine L,
- L'acide aminé en position 276 est la sérine S,
- L'acide aminé en position 277 est la tyrosine Y,
- L'acide aminé en position 287 est l'arginine R,
- L'acide aminé en position 310 est la proline P.

Ainsi, l'enzyme mutée obtenue comprend seulement les 7 mutations du groupe A, par rapport à la séquence SEQ ID NO : 1, et a donc pour séquence la séquence SEQ ID NO : 2.

Selon un autre mode de réalisation particulier, la demande décrit une enzyme PTE mutée comprenant les mutations du groupe A et comprenant au moins 2 mutations supplémentaires, en position 225 et 271.

Selon un autre mode de réalisation particulier, la demande décrit une enzyme PTE mutée de séquence SEQ ID NO : 3, comprenant les mutations du groupe A et dans laquelle les au moins 2 mutations supplémentaires ci-dessus sont les suivantes :
- En position 225 : substitution de l'histidine H par la tyrosine Y,
- En position 271 : substitution de la leucine L par la thréonine T.

Ainsi, l'enzyme mutée obtenue comprend les 7 mutations du groupe A et au moins les 2 mutations supplémentaires ci-dessus, sa séquence étant la séquence SEQ ID NO : 3.

Selon un autre mode de réalisation particulier, la demande décrit une enzyme PTE mutée comprenant les mutations du groupe A et comprenant au moins 8 mutations supplémentaires, en position 74, 100, 222, 225, 238, 240, 242 et 276.

Selon un autre mode de réalisation particulier, la demande décrit une enzyme PTE mutée de séquence SEQ ID NO : 4, comprenant les mutations du groupe A et dans laquelle les au moins 8 mutations supplémentaires ci-dessus sont les suivantes :
- En position 74 : substitution de l'isoleucine I par la cystéine,
- En position 100 : substitution de la phénylalanine F par la valine V,
- En position 222 : substitution de l'histidine H par la glutamine Q,
- En position 225 : substitution de l'histidine H par la tyrosine Y,
- En position 238 : substitution de l'alanine A par la valine V,
- En position 240 : substitution de la leucine L par la méthionine M,
- En position 242 : substitution de l'isoleucine I par l'asparagine N,
- En position 276 : substitution de la sérine S par la leucine L.

Ainsi, l'enzyme mutée obtenue comprend les 7 mutations du groupe A et au moins les 8 mutations supplémentaires ci-dessus, sa séquence étant la séquence SEQ ID NO : 4.

Selon un autre mode de réalisation particulier, la demande décrit une enzyme PTE mutée comprenant les mutations du groupe A et comprenant au moins 9 mutations supplémentaires, en position 45, 48, 100, 141, 153, 222, 242, 287 et 310.

Selon un autre mode de réalisation particulier, la demande décrit une enzyme PTE mutée de séquence SEQ ID NO : 5, comprenant les mutations du groupe A et dans laquelle les au moins 9 mutations supplémentaires ci-dessus sont les suivantes :
- En position 45 : substitution de la lysine K par l'alanine A,
- En position 48 : substitution de l'alanine A par la valine V,
- En position 100 : substitution de la phénylalanine F par l'acide glutamique E,
- En position 141 : substitution de la thréonine T par l'asparagine N,
- En position 153 : substitution de la lysine K par l'arginine R,
- En position 222 : substitution de l'histidine H par la glycine G,
- En position 242 : substitution de l'isoleucine I par l'asparagine N,
- En position 287 : substitution de l'arginine R par la sérine S,
- En position 310 : substitution de la proline P par la sérine S.

Ainsi, l'enzyme mutée obtenue comprend les 7 mutations du groupe A et au moins les 9 mutations supplémentaires ci-dessus, sa séquence étant la séquence SEQ ID NO : 5.

Selon un autre mode de réalisation particulier, la demande décrit une enzyme PTE mutée comprenant les mutations du groupe A et comprenant au moins 11 mutations supplémentaires, en position 45, 48, 100, 141, 153, 177, 201, 222, 242, 277 et 287.

Selon un autre mode de réalisation particulier, la demande décrit une enzyme PTE mutée de séquence SEQ ID NO : 6, comprenant les mutations du groupe A et dans laquelle les au moins 11 mutations supplémentaires ci-dessus sont les suivantes :
- En position 45 : substitution de la lysine K par l'alanine A,
- En position 48 : substitution de l'alanine A par la valine V,
- En position 100 : substitution de la phénylalanine F par l'acide glutamique E,
- En position 141 : substitution de la thréonine T par l'asparagine N,
- En position 153 : substitution de la lysine K par l'arginine R,
- En position 177 : substitution de la glycine G par l'acide aspartique D,
- En position 201 : substitution de l'acide aspartique D par la glycine G,
- En position 222 : substitution de l'histidine H par la glycine G,
- En position 242 : substitution de l'isoleucine I par l'asparagine N,
- En position 277 : substitution de la tyrosine Y par le tryptophane W,
- En position 287 : substitution de l'arginine R par la sérine S.

Ainsi, l'enzyme mutée obtenue comprend les 7 mutations du groupe A et au moins les 11 mutations supplémentaires ci-dessus, sa séquence étant la séquence SEQ ID NO : 6.

Selon un autre mode de réalisation particulier, la demande décrit une enzyme PTE mutée comprenant les mutations du groupe A et comprenant au moins 12 mutations supplémentaires, en position 45, 48, 74, 100, 141, 153, 177, 201, 222, 242, 277 et 287.

Selon un autre mode de réalisation particulier, la demande décrit une enzyme PTE mutée de séquence SEQ ID NO : 7, comprenant les mutations du groupe A et dans laquelle les au moins 12 mutations supplémentaires ci-dessus sont les suivantes :
- En position 45 : substitution de la lysine K par l'alanine A,
- En position 48 : substitution de l'alanine A par la valine V,
- En position 74 : substitution de l'isoleucine I par l'alanine A,
- En position 100 : substitution de la phénylalanine F par l'acide glutamique E,
- En position 141 : substitution de la thréonine T par l'asparagine N,
- En position 153 : substitution de la lysine K par l'arginine R,
- En position 177 : substitution de la glycine G par l'acide aspartique D,
- En position 201 : substitution de l'acide aspartique D par la glycine G,
- En position 222 : substitution de l'histidine H par la glycine G,
- En position 242 : substitution de l'isoleucine I par l'asparagine N,
- En position 277 : substitution de la tyrosine Y par le tryptophane W,
- En position 287 : substitution de l'arginine R par la sérine S.

Ainsi, l'enzyme mutée obtenue comprend les 7 mutations du groupe A et au moins les 12 mutations supplémentaires ci-dessus, sa séquence étant la séquence SEQ ID NO : 7.

Selon un autre mode de réalisation particulier, la demande décrit une enzyme PTE mutée comprenant les mutations du groupe A et comprenant au moins 13 mutations supplémentaires, en position 45, 48, 100, 141, 153, 177, 201, 222, 238, 239, 242, 277 et 287.

Selon un autre mode de réalisation particulier, la demande décrit une enzyme PTE mutée de séquence SEQ ID NO : 8, comprenant les mutations du groupe A et dans laquelle les au moins 13 mutations supplémentaires ci-dessus sont les suivantes :
- En position 45 : substitution de la lysine K par l'alanine A,
- En position 48 : substitution de l'alanine A par la méthionine M,
- En position 100 : substitution de la phénylalanine F par l'acide glutamique E,
- En position 141 : substitution de la thréonine T par l'asparagine N,
- En position 153 : substitution de la lysine K par l'arginine R,
- En position 177 : substitution de la glycine G par l'acide aspartique D,
- En position 201 : substitution de l'acide aspartique D par la glycine G,
- En position 222 : substitution de l'histidine H par la glycine G,
- En position 238 : substitution de l'alanine A par la sérine S,
- En position 239 : substitution de la leucine L par le tryptophane W,
- En position 242 : substitution de l'isoleucine I par l'asparagine N,
- En position 277 : substitution de la tyrosine Y par le tryptophane W,
- En position 287 : substitution de l'arginine R par la sérine S.

Ainsi, l'enzyme mutée obtenue comprend les 7 mutations du groupe A et au moins les 13 mutations supplémentaires ci-dessus, sa séquence étant la séquence SEQ ID NO : 8.

Selon un autre mode de réalisation particulier, la demande décrit une enzyme PTE mutée comprenant les mutations du groupe A et comprenant au moins 14 mutations supplémentaires, en position 45, 48, 74, 100, 141, 153, 177, 201, 222, 238, 239, 242, 277 et 287.

Selon un autre mode de réalisation particulier, la demande décrit une enzyme PTE mutée de séquence SEQ ID NO : 9, comprenant les mutations du groupe A et dans laquelle les au moins 14 mutations supplémentaires ci-dessus sont les suivantes :
- En position 45 : substitution de la lysine K par l'alanine A,
- En position 48 : substitution de l'alanine A par la méthionine M,
- En position 74 : substitution de l'isoleucine I par l'alanine A,
- En position 100 : substitution de la phénylalanine F par l'acide glutamique E,
- En position 141 : substitution de la thréonine T par l'asparagine N,
- En position 153 : substitution de la lysine K par l'arginine R,
- En position 177 : substitution de la glycine G par l'acide aspartique D,
- En position 201 : substitution de l'acide aspartique D par la glycine G,
- En position 222 : substitution de l'histidine H par la glycine G,
- En position 238 : substitution de l'alanine A par la sérine S,
- En position 239 : substitution de la leucine L par le tryptophane W,
- En position 242 : substitution de l'isoleucine I par l'asparagine N,
- En position 277 : substitution de la tyrosine Y par le tryptophane W,
- En position 287 : substitution de l'arginine R par la sérine S.

Ainsi, l'enzyme mutée obtenue comprend les 7 mutations du groupe A et au moins les 14 mutations supplémentaires ci-dessus, sa séquence étant la séquence SEQ ID NO : 9.

Selon un autre mode de réalisation particulier, la demande décrit une enzyme PTE mutée comprenant les mutations du groupe A et comprenant au moins 14 mutations supplémentaires, en position 45, 48, 100, 141, 153, 177, 201, 222, 235, 238, 239, 242, 277 et 287.

Selon un autre mode de réalisation particulier, la demande décrit une enzyme PTE mutée de séquence SEQ ID NO : 10, comprenant les mutations du groupe A et dans laquelle les au moins 14 mutations supplémentaires ci-dessus sont les suivantes :
- En position 45 : substitution de la lysine K par l'alanine A,
- En position 48 : substitution de l'alanine A par la méthionine M,
- En position 100 : substitution de la phénylalanine F par l'acide glutamique E,
- En position 141 : substitution de la thréonine T par l'asparagine N,
- En position 153 : substitution de la lysine K par l'arginine R,
- En position 177 : substitution de la glycine G par l'acide aspartique D,
- En position 201 : substitution de l'acide aspartique D par la glycine G,
- En position 222 : substitution de l'histidine H par la glycine G,
- En position 235 : substitution de la sérine S par la méthionine M,
- En position 238 : substitution de l'alanine A par la sérine S,
- En position 239 : substitution de la leucine L par le tryptophane W,
- En position 242 : substitution de l'isoleucine I par l'asparagine N,
- En position 277 : substitution de la tyrosine Y par le tryptophane W,
- En position 287 : substitution de l'arginine R par la sérine S.

Ainsi, l'enzyme mutée obtenue comprend les 7 mutations du groupe A et au moins les 14 mutations supplémentaires ci-dessus, sa séquence étant la séquence SEQ ID NO : 10.

Selon un autre mode de réalisation particulier, la demande décrit une enzyme PTE mutée comprenant les mutations du groupe A et comprenant au moins 15 mutations supplémentaires, en position 45, 48, 74, 100, 141, 153, 177, 201, 222, 235, 238, 239, 242, 277 et 287.

Selon un autre mode de réalisation particulier, la demande décrit une enzyme PTE mutée de séquence SEQ ID NO : 11, comprenant les mutations du groupe A et dans laquelle les au moins 15 mutations supplémentaires ci-dessus sont les suivantes :
- En position 45 : substitution de la lysine K par l'alanine A,
- En position 48 : substitution de l'alanine A par la méthionine M,
- En position 74 : substitution de l'isoleucine I par l'alanine A,
- En position 100 : substitution de la phénylalanine F par l'acide glutamique E,
- En position 141 : substitution de la thréonine T par l'asparagine N,
- En position 153 : substitution de la lysine K par l'arginine R,
- En position 177 : substitution de la glycine G par l'acide aspartique D,
- En position 201 : substitution de l'acide aspartique D par la glycine G,
- En position 222 : substitution de l'histidine H par la glycine G,
- En position 235 : substitution de la sérine S par la méthionine M,
- En position 238 : substitution de l'alanine A par la sérine S,
- En position 239 : substitution de la leucine L par le tryptophane W,
- En position 242 : substitution de l'isoleucine I par l'asparagine N,
- En position 277 : substitution de la tyrosine Y par le tryptophane W,
- En position 287 : substitution de l'arginine R par la sérine S.

Ainsi, l'enzyme mutée obtenue comprend les 7 mutations du groupe A et au moins les 15 mutations supplémentaires ci-dessus, sa séquence étant la séquence SEQ ID NO : 11.

Selon un autre mode de réalisation particulier, la demande décrit une enzyme PTE mutée de séquence SEQ ID NO : 12, comprenant les mutations du groupe A et dans laquelle les au moins 15 mutations supplémentaires ci-dessus sont les suivantes :
- En position 45 : substitution de la lysine K par l'alanine A,
- En position 48 : substitution de l'alanine A par la méthionine M,
- En position 74 : substitution de l'isoleucine I par la cystéine C,
- En position 100 : substitution de la phénylalanine F par l'acide glutamique E,
- En position 141 : substitution de la thréonine T par l'asparagine N,
- En position 153 : substitution de la lysine K par l'arginine R,
- En position 177 : substitution de la glycine G par l'acide aspartique D,
- En position 201 : substitution de l'acide aspartique D par la glycine G,
- En position 222 : substitution de l'histidine H par la glycine G,
- En position 235 : substitution de la sérine S par la méthionine M,
- En position 238 : substitution de l'alanine A par la sérine S,
- En position 239 : substitution de la leucine L par le tryptophane W,
- En position 242 : substitution de l'isoleucine I par l'asparagine N,
- En position 277 : substitution de la tyrosine Y par le tryptophane W,
- En position 287 : substitution de l'arginine R par la sérine S.

Ainsi, l'enzyme mutée obtenue comprend les 7 mutations du groupe A et au moins les 15 mutations supplémentaires ci-dessus, sa séquence étant la séquence SEQ ID NO : 12.

Selon un autre mode de réalisation particulier, la demande décrit une enzyme PTE mutée selon le groupe A de mutations, de séquence SEQ ID NO : 2, dans laquelle :
- L'acide aminé en position 45 est différent de l'alanine A,
- L'acide aminé en position 48 est différent de la valine V et de la méthionine M,
- L'acide aminé en position 74 est différent de la cystéine C et de l'alanine A,
- L'acide aminé en position 100 est différent de l'acide glutamique E et de la valine V,
- L'acide aminé en position 141 est différent de l'asparagine N,
- L'acide aminé en position 153 est différent de l'arginine R,
- L'acide aminé en position 177 est différent de l'acide aspartique D,
- L'acide aminé en position 201 est différent de la glycine G,
- L'acide aminé en position 222 est différent de la glycine G et de la glutamine Q,
- L'acide aminé en position 225 est différent de la tyrosine Y,
- L'acide aminé en position 235 est différent de la méthionine M,
- L'acide aminé en position 238 est différent de la valine V et de la sérine S,
- L'acide aminé en position 239 est différent du tryptophane W,
- L'acide aminé en position 240 est différent de la méthionine M,
- L'acide aminé en position 242 est différent de l'asparagine N,
- L'acide aminé en position 271 est différent de la thréonine T,
- L'acide aminé en position 276 est différent de la leucine L,
- L'acide aminé en position 277 est différent du tryptophane W,
- L'acide aminé en position 287 est différent de la sérine S,
- L'acide aminé en position 310 est différent de la sérine S.

Selon un mode de réalisation particulièrement préféré, la demande décrit une enzyme PTE de séquences SEQ ID NO : 3, de séquence SEQ ID NO : 4 ou de séquence SEQ ID NO : 5.

Un aspect de la présente demande décrit l'utilisation d'au moins 7 mutations, notamment de 7 mutations, pour augmenter la stabilité d'une enzyme phosphotriestérase (PTE) de séquence SEQ ID NO : 1 capable d'hydrolyser les composés organophosphorés, en substituant dans la séquence SEQ ID NO : 1 :
l'acide aminé en position 13 par la proline P,
l'acide aminé en position 14 par la valine V,
l'acide aminé en position 60 par la sérine S,
l'acide aminé en position 79 par l'arginine R,
l'acide aminé en position 124 par l'histidine H,
l'acide aminé en position 218 par la valine V,
l'acide aminé en position 258 par l'arginine R,
afin d'obtenir une enzyme PTE mutée, notamment de séquence SEQ ID NO : 2, qui possède une stabilité améliorée en comparaison de la stabilité de l'enzyme de séquence SED ID NO : 1.

Comme vu précédemment, les 7 mutations aux positions 13, 14, 60, 79, 124, 218, 258 constituent les mutations du groupe A.

Selon un autre mode de réalisation particulier, la demande décrit l'utilisation d'au moins les 7 mutations du groupe A, combinées à des mutations supplémentaires pour augmenter la stabilité et l'activité catalytique phosphotriestérase (PTE) de l'enzyme de séquence SEQ ID NO : 1 capable d'hydrolyser les composés organophosphorés, en effectuant dans la séquence SEQ ID NO : 1 au moins deux substitutions supplémentaires choisies parmi les substitutions de :
l'acide aminé en position 45 par l'alanine A,
l'acide aminé en position 48 par la valine V ou la méthionine M,
l'acide aminé en position 74 par la cystéine C ou l'alanine A,
l'acide aminé en position 100 par l'acide glutamique E ou la valine V,
l'acide aminé en position 141 par l'asparagine N,
l'acide aminé en position 153 par l'arginine R,
l'acide aminé en position 177 par l'acide aspartique D,
l'acide aminé en position 201 par la glycine G,
l'acide aminé en position 222 par la glycine G ou la glutamine Q,
l'acide aminé en position 225 par la tyrosine Y,
l'acide aminé en position 235 par la méthionine M,
l'acide aminé en position 238 par la valine V ou la sérine S,
l'acide aminé en position 239 par le tryptophane W,
l'acide aminé en position 240 par la méthionine M,
l'acide aminé en position 242 par l'asparagine N,
l'acide aminé en position 271 par la thréonine T,
l'acide aminé en position 276 par la leucine L,
l'acide aminé en position 277 par le tryptophane W,
l'acide aminé en position 287 par la sérine S,
l'acide aminé en position 310 par la sérine S.
afin d'obtenir une enzyme PTE mutée qui possède une stabilité améliorée et une activité catalytique d'hydrolyse des composés organophosphorés améliorée, en comparaison de la stabilité et de l'activité catalytique de l'enzyme de séquence SED ID NO : 1.

Par « augmentation de l'activité catalytique » ou « activité catalytique améliorée », on désigne une augmentation supérieure à un facteur 10 du rapport K_{cat}/Kₘ de l'enzyme mutée par rapport à celui de la parathion hydrolase sauvage, de séquence SEQ ID NO : 1.

A titre d'exemple, une enzyme mutée ayant un K_{cat}/Kₘ = 6.10⁶ M⁻¹.s⁻¹ sera considérée comme ayant une activité catalytique améliorée par rapport à une enzyme ayant un K_{cat}/Kₘ = 5.10⁵ M⁻¹.s⁻¹.

Selon un autre mode de réalisation particulier, l'enzyme PTE est mutée par l'utilisation d'au moins les 7 mutations du groupe A, combinées à des mutations supplémentaires pour augmenter l'activité catalytique phosphotriestérase (PTE) de l'enzyme de séquence SEQ ID NO : 1 capable d'hydrolyser les composés organophosphorés, en effectuant dans la séquence SEQ ID NO : 1 au moins 2 substitutions supplémentaires afin d'obtenir une enzyme mutée de séquence choisie parmi les suivantes : SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12.

Dans un mode de réalisation particulièrement préféré, l'enzyme PTE est mutée par l'utilisation d'au moins les 7 mutations du groupe A, combinées à des mutations supplémentaires pour augmenter l'activité catalytique phosphotriestérase (PTE) de l'enzyme de séquence SEQ ID NO : 1 capable d'hydrolyser les composés organophosphorés, en effectuant dans la séquence SEQ ID NO : 1 au moins 2 substitutions supplémentaires afin d'obtenir une enzyme mutée de séquence choisie parmi les suivantes : SEQ ID NO : 3, SEQ ID NO : 4, SEQ ID NO : 5.

Ainsi, l'enzyme PTE mutée obtenue possède une stabilité améliorée et une activité catalytique d'hydrolyse des composés organophosphorés améliorée, en comparaison de la stabilité et de l'activité catalytique de l'enzyme de séquence SED ID NO : 1.

Un mode de réalisation particulier concerne l'utilisation d'au moins les 7 mutations du groupe A, en combinaison avec 8 mutations supplémentaires pour augmenter la stabilité et l'activité catalytique phosphotriestérase (PTE) de l'enzyme de séquence SEQ ID NO : 1 capable d'hydrolyser les composés organophosphorés, lesdites 8 mutations supplémentaires consistant en la substitution dans la séquence SEQ ID NO : 1 de :
l'acide aminé en position 74 par la cystéine C,
l'acide aminé en position 100 par la valine V,
l'acide aminé en position 222 par la glutamine Q,
l'acide aminé en position 225 par la tyrosine Y,
l'acide aminé en position 238 par la valine V,
l'acide aminé en position 240 par la méthionine M,
l'acide aminé en position 242 par l'asparagine N,
l'acide aminé en position 276 par la leucine L,
afin d'obtenir une enzyme PTE mutée de séquence SEQ ID NO : 4 qui possède une stabilité améliorée et une activité catalytique d'hydrolyse des composés organophosphorés améliorée en comparaison de la stabilité et de l'activité catalytique de l'enzyme de séquence SED ID NO : 1.

Dans un aspect de la demande, les composés OP hydrolysés par les enzymes PTE mutées de la présente demande peuvent être des insecticides organophosphorés et des organophosphorés des armes chimiques.

Dans un aspect particulier de la présente demande, les insecticides organophosphorés hydrolysés par les enzymes PTE de la présente demande peuvent être l'un des éléments suivants :

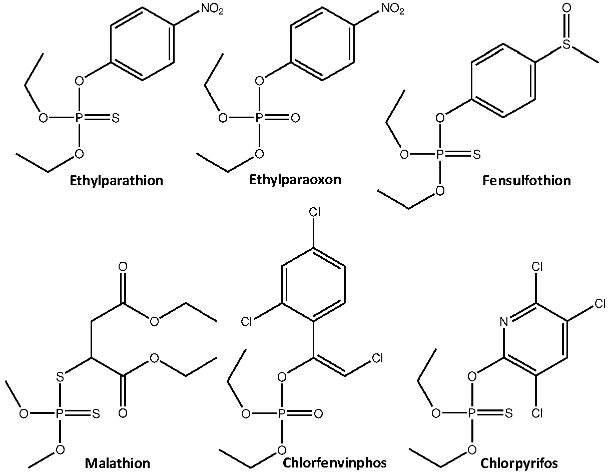

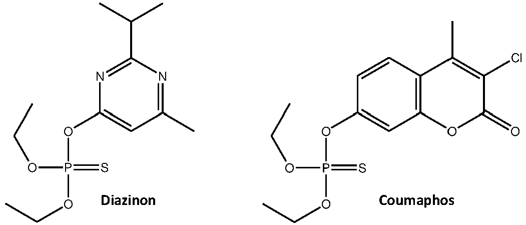

Dans un aspect particulier de la présente demande, les organophosphorés des armes chimiques hydrolysés par les enzymes PTE de la présente demande peuvent être l'un des éléments suivants :

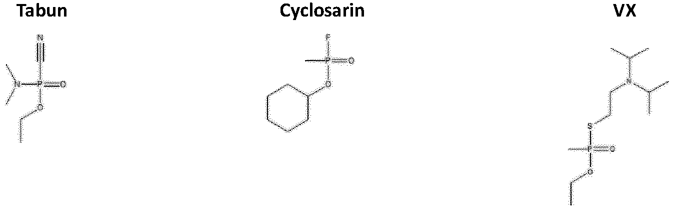

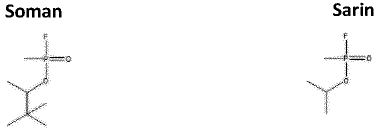

Dans un aspect particulièrement préféré, la demande décrit l'enzyme PTE mutée de séquence SEQ ID NO : 3, ladite enzyme PTE mutée de séquence SEQ ID NO : 3 étant capable d'hydrolyser les insecticides organophosphorés suivants :

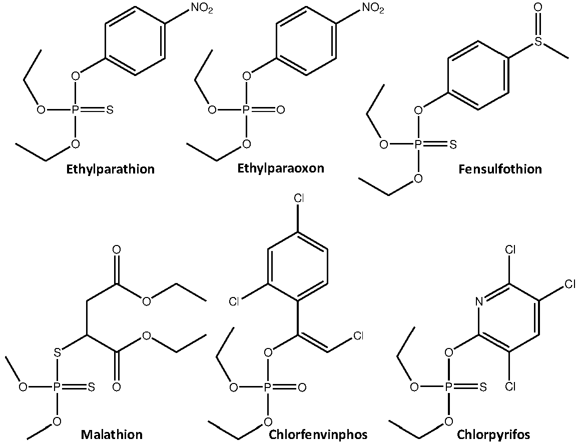

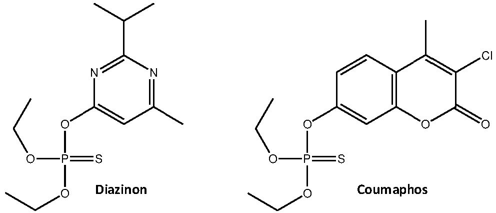

Dans un aspect particulièrement préféré, la demande décrit l'enzyme PTE mutée de séquence SEQ ID NO : 3, ladite enzyme PTE mutée de séquence SEQ ID NO : 3 étant capable d'hydrolyser les organophosphorés des armes chimiques suivants :

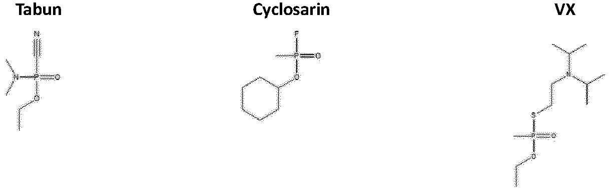

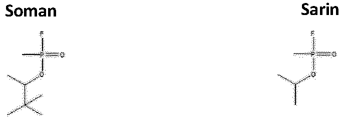

Dans un aspect particulièrement préféré, la demande décrit l'enzyme PTE mutée de séquence SEQ ID NO : 4, ladite enzyme PTE mutée de séquence SEQ ID NO : 4 étant capable d'hydrolyser les insecticides organophosphorés suivants :

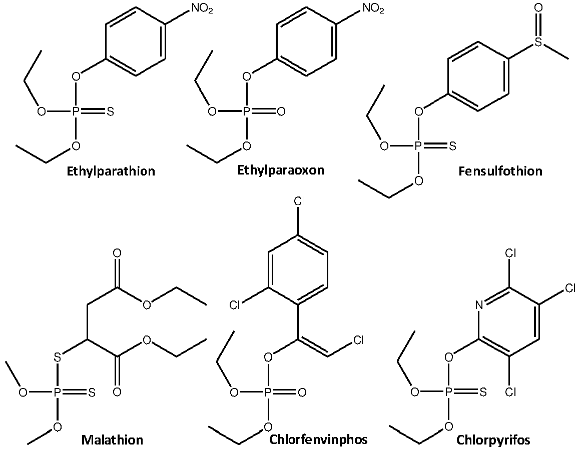

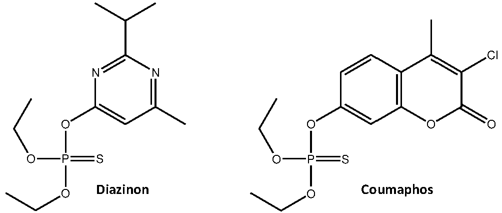

Dans un aspect particulièrement préféré, la demande décrit l'enzyme PTE mutée de séquence SEQ ID NO : 4, ladite enzyme PTE mutée de séquence SEQ ID NO : 4 étant capable d'hydrolyser les organophosphorés des armes chimiques suivants :

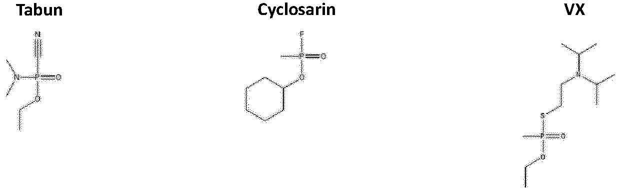

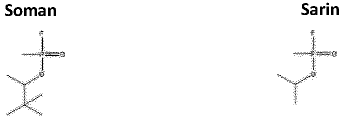

Dans un aspect particulièrement préféré, la demande décrit l'enzyme PTE mutée de séquence SEQ ID NO : 5, ladite enzyme PTE mutée de séquence SEQ ID NO : 5 étant capable d'hydrolyser les insecticides organophosphorés suivants :

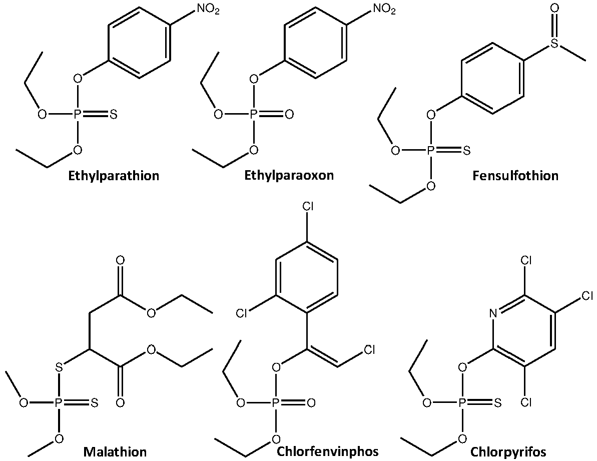

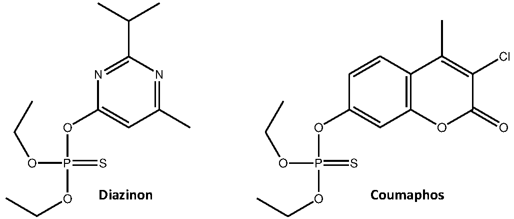

Dans un aspect particulièrement préféré, la demande décrit l'enzyme PTE mutée de séquence SEQ ID NO : 5, ladite enzyme PTE mutée de séquence SEQ ID NO : 5 étant capable d'hydrolyser les organophosphorés des armes chimiques suivants :

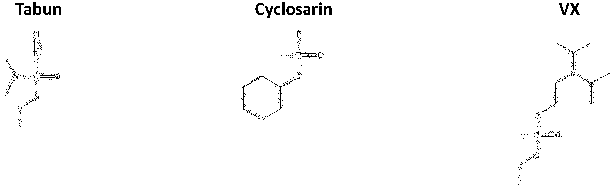

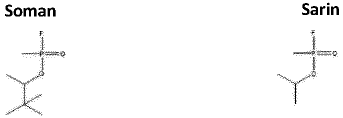

Un autre aspect de la présente demande décrit l'utilisation d'au moins une enzyme PTE mutée telle que définie ci-dessus, possédant une activité catalytique phosphotriestérase (PTE) capable d'hydrolyser les composés organophosphorés :
- pour la décontamination de sols pollués par des composés organophosphorés, ou
- pour la décontamination d'une surface, de la peau, des muqueuses ou des cheveux contaminés avec des composés organophosphorés, ou
- pour la prévention ou du traitement d'un empoisonnement interne ou externe par ingestion ou inhalation d'un composé organophosphoré, ou
- pour le contrôle de la pollution des eaux polluées par des composés organophosphorés, ou
- pour la destruction de stocks d'agents neurotoxiques, ou
- pour la décontamination de textiles et filtres, ou
- pour la décontamination de peintures,
ladite au moins une enzyme PTE mutée étant préférentiellement choisie parmi les enzymes mutées de séquence SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12, seules ou en combinaisons entre elles.

Un mode de réalisation particulièrement préféré de la présente demande décrit l'utilisation d'au moins une enzyme PTE mutée de séquence SEQ ID NO : 3, SEQ ID NO : 4 ou SEQ ID NO : 5, possédant une activité catalytique phosphotriestérase (PTE) capable d'hydrolyser les composés organophosphorés :
- pour la décontamination de sols pollués par des composés organophosphorés, ou
- pour la décontamination d'une surface, de la peau, des muqueuses ou des cheveux contaminés avec des composés organophosphorés, ou
- pour la prévention ou du traitement d'un empoisonnement interne ou externe par ingestion ou inhalation d'un composé organophosphoré, ou
- pour le contrôle de la pollution des eaux polluées par des composés organophosphorés, ou
- pour la destruction de stocks d'agents neurotoxiques, ou
- pour la décontamination de textiles et filtres, ou
- pour la décontamination de peintures.

Un autre aspect de la présente demande décrit un kit pour la décontamination de surfaces, de la peau ou des muqueuses, des cheveux, de peintures, de tissus ou de filtres contaminés avec des composés organophosphorés, ledit kit comprenant au moins une enzyme PTE mutée telle que définie ci-dessus, ayant une activité de catalyse des composés organophosphorés et ladite au moins une enzyme mutée étant préférentiellement choisie parmi les enzymes mutées de séquence SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12, seules ou en combinaisons entre elles.

Un mode de réalisation particulièrement préféré de la présente demande décrit un kit pour la décontamination de surfaces, de la peau ou des muqueuses, des cheveux, de peintures, de tissus ou de filtres contaminés avec des composés organophosphorés, ledit kit comprenant au moins une enzyme PTE mutée de séquence SEQ ID NO : 3, SEQ ID NO : 4 ou SEQ ID NO : 5 ayant une activité de catalyse des composés organophosphorés, seules ou en combinaisons entre elles.

Un autre aspect de la présente demande décrit une composition phytosanitaire comprenant comme principe actif au moins une enzyme PTE mutée telle que définie ci-dessus, possédant une activité de catalyse des composés organophosphorés, ladite au moins une enzyme mutée étant préférentiellement choisie parmi les enzymes mutées de séquence SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12, seules ou en combinaisons entre elles.

Un mode de réalisation particulièrement préféré de la présente demande décrit une composition phytosanitaire comprenant comme principe actif au moins une enzyme PTE mutée de séquence SEQ ID NO : 3, SEQ ID NO : 4 ou SEQ ID NO : 5, possédant une activité de catalyse des composés organophosphorés, seules ou en combinaisons entre elles.

Un autre aspect de la présente demande décrit une composition pharmaceutique comprenant comme principe actif au moins une enzyme PTE mutée telle que définie ci-dessus, possédant une activité de catalyse des composés organophosphorés, ladite au moins une enzyme mutée étant préférentiellement choisie parmi les enzymes mutées de séquence SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12, seules ou en combinaisons entre elles ; en combinaison d'un excipient pharmaceutiquement acceptable.

Un mode de réalisation particulièrement préféré de la présente demande décrit une composition pharmaceutique comprenant comme principe actif au moins une enzyme PTE mutée de séquence SEQ ID NO : 3, SEQ ID NO : 4 ou SEQ ID NO : 5, possédant une activité de catalyse des composés organophosphorés, seules ou en combinaisons entre elles ; en combinaison d'un excipient pharmaceutiquement acceptable.

Un autre aspect de la présente demande décrit une enzyme PTE mutée telle que définie ci-dessus, possédant une activité de catalyse des composés organophosphorés, ladite au moins une enzyme mutée étant préférentiellement choisies parmi les enzymes mutées de séquence SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12, seules ou en combinaisons entre elles, pour son utilisation dans le traitement ou la prévention des empoisonnements par contact, inhalation ou ingestion de composés organophosphorés.

Un mode de réalisation particulièrement préféré de la présente demande décrit une enzyme PTE mutée de séquence SEQ ID NO : 3, SEQ ID NO : 4 ou SEQ ID NO : 5, possédant une activité de catalyse des composés organophosphorés, seules ou en combinaisons entre elles, pour son utilisation dans le traitement ou la prévention des empoisonnements par contact, inhalation ou ingestion de composés organophosphorés.
Un autre aspect de la présente demande décrit une méthode de traitement des empoisonnements par contact, inhalation ou ingestion de composés organophosphorés comprenant l'administration d'au moins une enzyme PTE mutées telle que définie ci-dessus, possédant une activité de catalyse des composés organophosphorés, ladite au moins une enzyme mutée étant préférentiellement choisies parmi les enzymes mutées de séquence SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12, seules ou en combinaisons entre elles
Un mode de réalisation particulièrement préféré de la présentedemande décrit une méthode de traitement des empoisonnements par contact, inhalation ou ingestion de composés organophosphorés comprenant l'administration d'au moins une enzyme PTE_mutées de séquence SEQ ID NO : 3, SEQ ID NO : 4 ou SEQ ID NO : 5, possédant une activité de catalyse des composés organophosphorés, seules ou en combinaisons entre elles
Un autre aspect de la présente demande décrit une méthode de prévention des empoisonnements par contact, inhalation ou ingestion de composés organophosphorés comprenant l'administration d'au moins une enzyme PTE mutées telle que définie ci-dessus, possédant une activité de catalyse des composés organophosphorés, ladite au moins une enzyme mutée étant préférentiellement choisies parmi les enzymes mutées de séquence SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11 ou SEQ ID NO : 12, seules ou en combinaisons entre elles
Un mode de réalisation particulièrement préféré de la présente demande décrit une méthode de prévention des empoisonnements par contact, inhalation ou ingestion de composés organophosphorés comprenant l'administration d'au moins une enzyme PTE mutées de séquence SEQ ID NO : 3, SEQ ID NO : 4 ou SEQ ID NO : 5, possédant une activité de catalyse des composés organophosphorés, seules ou en combinaisons entre elles

### Légende des figures :

La figure 1 représente l'évolution du dichroïsme circulaire (CD) en fonction de la température pour l'enzyme de séquence SEQ ID NO : 13.
La figure 2 représente l'évolution du dichroïsme circulaire (CD) en fonction de la température pour l'enzyme de séquence SEQ ID NO : 4.

### Matériels & méthodes

### 1. Production des enzymes

Les gènes codant pour chaque enzyme ont été optimisés pour l'expression au sein d*'E.Coli* et synthétisés par GeneScript puis insérés dans le plasmide pET22b en utilisant les enzymes de restriction Ndel et Notl.

La production de la protéine a été réalisée au sein d*'E.Coli* BL21 (DE₃)-pGro7/GroEL dans 2 litres de milieu ZYP (Tryptone 10 g/L, Extrait de levure 5 g/L, (NH₄)₂SO₄ 66 g/L, KH₂PO₄ 136 g/L, Na₂HPO₄ 142 g/L, Glycérol 250 g (p/v), Glucose 25 g, α-lactose 100g, 100µg/ml ampicilline et 34 µg/ml chloramphénicol) inoculée en pré-culture sur une nuit avec un ratio 1/100.

La croissance se déroule à 37°C jusqu'à atteindre une DO₆₀₀ₙₘ=0,8. L'induction est réalisée par ajout de L-arabinose 0,2% au milieu ZYP pour chaque PTE ainsi que CoCl₂ 0,2mM pour les PTEs de séquence SEQ ID NO : 3 et SEQ ID NO : 4 et ZnCl₂ 0,1 mM pour la PTE de séquence SEQ ID NO : 5 et un changement de température de 16°C pendant 20 heures.

Les cellules sont ensuite récoltées par centrifugation (6420g, 30 min, 4°C), puis re-suspendues dans le tampon de lyse (Tris 50 mM pH 8, NaCl 300 mM, DNAsel 10 µg/mL, lysozyme 0,25 mg/mL, PMSF 0,1 mM) pendant 4 heures à température ambiante et enfin stockées à -80°C sur la nuit.

Les cellules subissent une sonication (3 étapes de 30 secondes) pour la lyse mécanique (Amplitude 45, time 00:30, pulse on 00:01, pulse off 00:01). Les débris cellulaires sont finalement éliminés par centrifugation (11000 rpm, 20 min, 4°C). Avant de passer à l'étape de purification, une filtration à 0,8 µm est nécessaire.

La purification se fait par chromatographie d'affinité Strep-Tag (StrepTrap^{™} HP 5ml). Le lavage ainsi que l'équilibration de la colonne se fait avec le tampon PTE (50 mM Tris, 300 mM NaCl pH 8) alors que l'élution de l'échantillon se fait avec le tampon 50 mM Tris, 300 mM NaCl, 2.5 mM desthiobiotin, pH 8 pour un débit de 2ml/min.

### 3. Mesure de la stabilité

### Détermination de la température de fusion

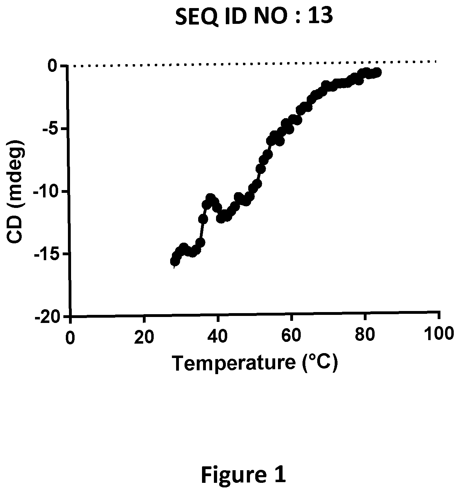

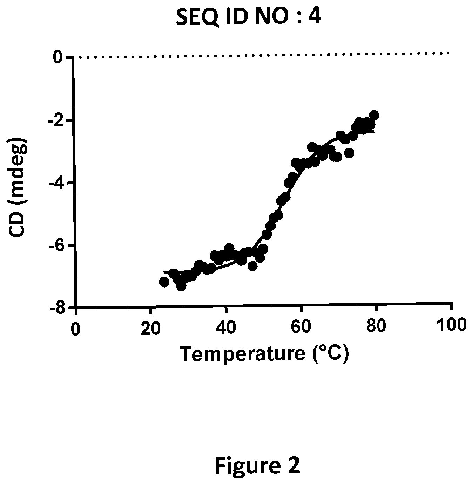

Le spectre de dichroïsme circulaire a été obtenu en utilisant un spectrometer Jasco J-815 CD avec un système de contrôle de la température de type Pelletier (Jasco MPTC-4905) dans une cellule à quartz Starna^{®} de 1 mm d'épaisseur et en utilisant le logiciel Spectra Manager. Les expériences ont été réalisées dans du tampon Tris 50 mM à pH 8. Les concentrations en protéines étant de l'ordre de 0.1 - 0,2 mg/mL, la dénaturation a été effectuée à 222 nm avec une augmentation de la température de 25 à 85°C (à 5°C/min). Les données ont été analysées avec GraphPad Prism 6, en utilisant l'équation sigmoïde de Boltzmann. Les résultats sont visibles sur les figures 1 et 2 ainsi que dans le Tableau 1 qui compare la température de fusion pour l'enzyme de séquence SEQ ID NO : 4 à celle de l'enzyme de séquence SEQ ID NO : 13 (influence de la présence des mutations ancestrales sur la stabilité de l'enzyme).

**Tableau 1 : Température de fusion (Tm) en fonction de la présence ou de l'absence des mutations ancestrales. La présence des mutations ancestrales (SEQ ID NO : 4) engendre une augmentation de la température de fusion de l'enzyme, et donc sa stabilité par rapport à l'enzyme dépourvue desdites mutations ancestrales (SEQ ID NO : 13)**

| **PTE** | **Tm (°C)** |
|---|---|
| **SEQ ID NO : 13** | **52,01** |
| **SEQ ID NO : 4** | **55,64** |

### 4. Mesure de l'activité

### Détermination de l'activité sur l'éthyl-paraoxon de formule :

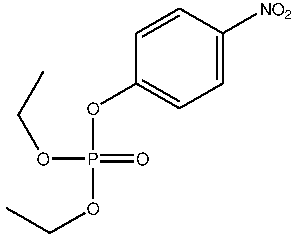

Les données ont été analyses avec GraphPad Prism 6, en utilisant la modélisation de type « one phase decay ». Les résultats sont exprimés au sein du Tableau 2 qui compare les valeurs des rapports Kcat / Km pour les enzymes de séquence SEQ ID NO : 4 et SEQ ID NO : 13 vis-à-vis de l'éthyl-paraoxon (influence de la présence des mutations ancestrales sur l'activité catalytique de l'enzyme vis-à-vis de ce substrat).

### Tampon PTE : Tris 50 mM, NaCl 300 mM, CoCl₂ 100µM, pH 8.

**Tableau 2 : Activité catalytique envers l'ethyl-paraoxon de l'enzyme de séquence SEQ ID NO : 13 et SEQ ID NO : 4.**

| **PTE** | **k_{cat}/k_{M} (M⁻¹.s⁻¹)** |
|---|---|
| **SEQ ID NO : 13** | **6.10⁵** |
| **SEQ ID NO : 4** | **3.10⁵** |

### Détermination de l'activité sur le DEVX de formule :

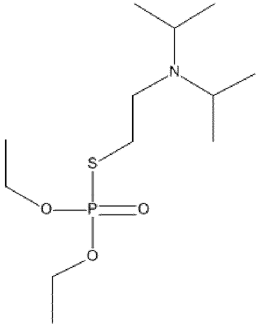

Les données ont été analysées avec GraphPad Prism 6, en utilisant une modélisation selon l'équation de Michaelis-Menten. Les résultats sont exprimés au sein du Tableau 3 qui compare les valeurs des Kcat, Km et des rapports Kcat / Km pour les enzymes de séquence SEQ ID NO : 4 et SEQ ID NO : 13 vis-à-vis du DEVX (influence de la présence des mutations ancestrales sur l'activité catalytique de l'enzyme vis-à-vis de ce substrat).

### Tampon : NaCl 300mM, TRIS 50mM, CoCl₂ 100µM, DTNB 4mM, pH8.

**Tableau 3 : Activité catalytique envers le DEVX de l'enzyme de séquence SEQ ID NO : 13 et SEQ ID NO : 4.**

| **PTE** | **k_{cat} (s⁻¹)** | **k_{M} (M)** | **k_{cat}/k_{M} (M⁻¹.s⁻¹)** |
|---|---|---|---|
| **SEQ ID NO : 13** | **3,356** | **0,001767** | **≈ 1,9.10³** |
| **SEQ ID NO : 4** | **3.10⁵** | **0,001317** | **≈ 1,3.10³** |

### 5. Mesure de l'activité des PTE mutées de la demande sur des insecticides organophosphorés

Les paramètres catalytiques des PTE mutées de la demande, notamment des PTE mutées de séquences SEQ ID NO : 3, SEQ ID NO : 4 ou SEQ ID NO : 5, sont mesurés à 25 °C en triplicat dans des plaques de 96 puits avec un volume réactionnel de 200 µL et enregistrés par un lecteur de microplaques (Synergy HT, BioTek, USA) dans une cellule de 6,2 mm en utilisant le logiciel Gen5.1.

Les dosages cinétiques sont effectués à des concentrations d'insecticides organophosphorés comprises entre 0,05 et 2 mM. L'efficacité d'hydrolyse des insecticides organophosphorés par les PTE mutées de la demande est déterminée en mesurant l'absorbance ou la fluorescence pendant 10 min grâce à un lecteur de microplaque. L'efficacité catalytique kcat/KM est alors déterminée. Les dosages cinétiques sont effectués dans du tampon d'activité (HEPES 50 mM ou Tris pH 8,0, NaCl 150 mM). Les paramètres catalytiques sont obtenus en ajustant les données à l'équation de Michaelis-Menten (MM).

Les insecticides organophosphorés utilisés pour mesurer l'activité des PTE mutées de la demande sont les suivants :

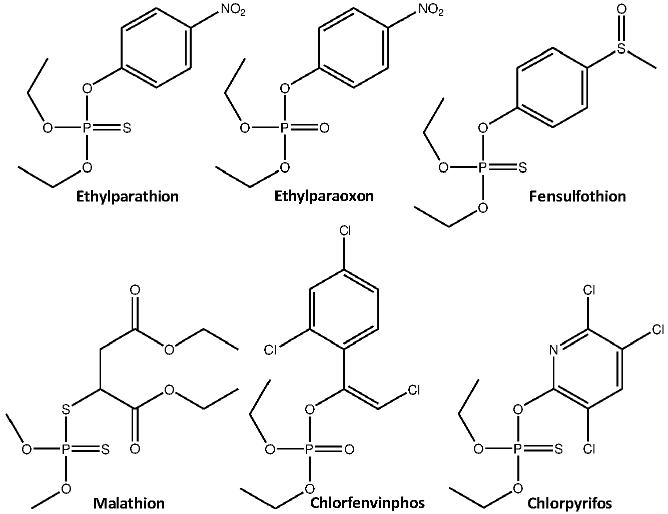

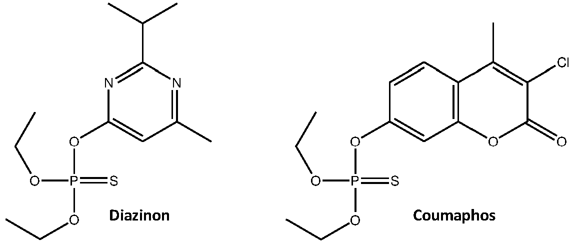

### 6. Mesure de l'activité des PTE mutées de la demande sur des organophosphorés d'armes chimiques

### a) Cinétique de dégradation et analyse RMN:

La dégradation des organophosphorés d'armes chimiques (CWNA), notamment du Soman et du VX, par les enzymes PTE mutées de la demande, notamment des PTE mutées de séquences SEQ ID NO : 3, SEQ ID NO : 4 ou SEQ ID NO : 5, est surveillée au fil du temps par RMN 31P.

Jusqu'à 10 µl de CWNA sont placés dans un tube RMN et 0,6 à 1,0 mL d'enzyme PTE mutées de la demande, notamment des PTE mutées de séquences SEQ ID NO : 3, SEQ ID NO : 4 ou SEQ ID NO : 5, ou d'eau (contrôle) sont ajoutés. L'enregistrement du signal est effectué à température ambiante (environ 23°C) et les signaux sont mesurés toutes les 10 minutes pendant 1 heure.

L'efficacité de la dégradation des CWNA est déterminée en mesurant l'intégrale des produits sous la forme de la fraction de la somme de toutes les intégrales du 31P. Les valeurs obtenues sont rapportées comme l'écart-type de la moyenne de deux mesures de dégradation effectuées séparément.

### b) Décontamination des surfaces :

L'efficacité de la décontamination de surface contaminées avec des CWNA, notamment du Soman et du VX, par les PTE mutées de la demande, notamment des PTE mutées de séquences SEQ ID NO : 3, SEQ ID NO : 4 ou SEQ ID NO : 5, est évaluée au fil du temps

Les panneaux de matériaux (5x5 cm) sont imprégnés de CWNA de façon à atteindre une contamination de 10 g/m2. Ensuite, les panneaux sont immergés dans une solution contenant les PTE de la demande, notamment des PTE mutées de séquences SEQ ID NO : 3, SEQ ID NO : 4 ou SEQ ID NO : 5, pendant une durée allant de 15 minutes à 1 heure.

Les panneaux sont ensuite lavés avec de l'eau et soigneusement séchés avec une lingette sans frotter. Tout agent CWNA résiduel sur le panneau est ensuite extrait avec un solvant approprié et analysé et quantifié par chromatographie en phase gazeuse couplée à la spectrométrie de masse (GC-MS).

Les organophosphorés d'armes chimiques utilisés pour mesurer l'activité des PTE mutées de la demande et leur capacité à décontaminer des surfaces imprégnées sont les suivants :

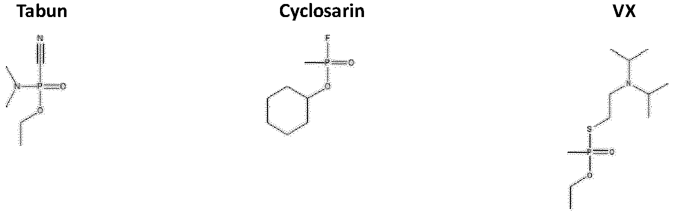

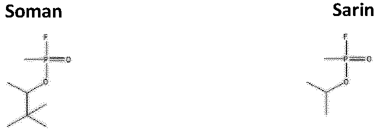

## Revendications

1. - Enzyme PTE mutée dérivée de la parathion hydrolase ayant la séquence SEQ ID NO : 1 et contenant les mutations suivantes par rapport à la séquence SEQ ID NO : 1:
- Substitution de la thréonine T par la proline P, en position 13,
- Substitution de l'isoleucine I par la valine V, en position 14,
- Substitution de l'alanine A par la sérine S, en position 60,
- Substitution de la sérine S par l'arginine R, en position 79,
- Substitution de la tyrosine Y par l'histidine H, en position 124,
- Substitution de l'isoleucine I par la valine V, en position 218,
- Substitution de l'histidine H par la tyrosine Y, en position 225
- Substitution de la glutamine Q par l'arginine R, en position 258
- Substitution de la leucine L par la thréonine T, en position 271 ladite enzyme PTE mutée étant de séquence SED ID NO : 3, ou contenant les mutations suivantes par rapport à la séquence SEQ ID NO : 1:
- Substitution de la thréonine T par la proline P, en position 13,
- Substitution de l'isoleucine I par la valine V, en position 14,
- Substitution de l'alanine A par la sérine S, en position 60,
- Substitution de l'isoleucine I par la cystéine C, en position 74
- Substitution de la sérine S par l'arginine R, en position 79,
- Substitution de la phénylalanine F par la valine V, en position 100
- Substitution de la tyrosine Y par l'histidine H, en position 124,
- Substitution de l'isoleucine I par la valine V, en position 218,
- Substitution de l'histidine H par la glutamine Q, en position 222
- Substitution de l'histidine H par la tyrosine Y, en position 225
- Substitution de l'alanine A par la valine V, en position 238
- Substitution de la leucine L par la méthionine M, en position 240
- Substitution de l'isoleucine I par l'asparagine N, en position 242
- Substitution de la glutamine Q par l'arginine R, en position 258
- Substitution de la sérine S par la leucine L, en position 276,
ladite enzyme PTE mutée étant de séquence SED ID NO : 4.

2. - Procédé d'obtention d'une enzyme PTE mutée comprenant l'introduction de 9 mutations, pour augmenter l'activité catalytique phosphotriestérase (PTE) de l'enzyme de séquence SEQ ID NO : 1 capable d'hydrolyser les composés organophosphorés, en substituant dans la séquence SEQ ID NO : 1 :
- l'acide aminé en position 13 par la proline P,
- l'acide aminé en position 14 par la valine V,
- l'acide aminé en position 60 par la sérine S,
- l'acide aminé en position 79 par l'arginine R,
- l'acide aminé en position 124 par l'histidine H,
- l'acide aminé en position 218 par la valine V,
- l'acide aminé en position 258 par l'arginine R,
- l'acide aminé en position 225 par tyrosine Y
- l'acide aminé en position 271 par la thréonine T
afin d'obtenir une enzyme PTE mutée de séquence SEQ ID NO : 3,
ou comprenant l'introduction de 15 mutations, pour augmenter l'activité catalytique phosphotriestérase (PTE) de l'enzyme de séquence SEQ ID NO : 1 capable d'hydrolyser les composés organophosphorés, en substituant dans la séquence SEQ ID NO : 1 :
- l'acide aminé en position 13 par la proline P,
- l'acide aminé en position 14 par la valine V,
- l'acide aminé en position 60 par la sérine S,
- l'acide aminé en position 74 par la cystéine
- l'acide aminé en position 79 par l'arginine R,
- l'acide aminé en position 100 par la valine V,
- l'acide aminé en position 124 par l'histidine H,
- l'acide aminé en position 218 par la valine V,
- l'acide aminé en position 222 par la glutamine Q,
- l'acide aminé en position 225 par la tyrosine Y,
- l'acide aminé en position 238 par la valine V,
- l'acide aminé en position 240 par la méthionine M,
- l'acide aminé en position 242 par l'asparagine N,
- l'acide aminé en position 258 par l'arginine R,
- l'acide aminé en position 276 par la leucine L
afin d'obtenir une enzyme PTE mutée de séquence SEQ ID NO : 4,
lesdites séquences SEQ ID NO : 3 et SEQ ID NO : 4 possèdent une activité catalytique d'hydrolyse des composés organophosphorés améliorée en comparaison de l'activité catalytique de l'enzyme de séquence SED ID NO : 1.

3. - Utilisation d'au moins une enzyme PTE mutée selon la revendication 1, et possédant une activité catalytique phosphotriestérase (PTE) capable d'hydrolyser les composés organophosphorés :
- pour la décontamination de sols pollués par des composés organophosphorés, ou
- pour la décontamination d'une surface, de la peau, des muqueuses ou des cheveux contaminés avec des composés organophosphorés, ou
- pour la prévention ou du traitement d'un empoisonnement interne ou externe par ingestion ou inhalation d'un composé organophosphoré, ou
- pour le contrôle de la pollution des eaux polluées par des composés organophosphorés, ou
- pour la destruction de stocks d'agents neurotoxiques,
ladite au moins une enzyme PTE mutée étant choisie parmi les enzymes mutées de séquence SEQ ID NO : 3 ou SEQ ID NO : 4, seules ou en combinaisons entre elles.

4. - Kit pour la décontamination de surfaces, de la peau ou des muqueuses, des cheveux, contaminés avec des composés organophosphorés, de tissus, de filtres ou de peintures, ledit kit comprenant au moins une enzyme PTE mutée selon la revendication 1, ayant une activité de catalyse des composés organophosphorés et ladite au moins une enzyme mutée étant choisie parmi les enzymes mutées de séquence SEQ ID NO : 3 ou SEQ ID NO : 4, seules ou en combinaisons entre elles.

5. - Composition phytosanitaire comprenant comme principe actif au moins une enzyme PTE mutée selon la revendication 1, et possédant une activité de catalyse des composés organophosphorés, ladite au moins une enzyme mutée étant choisie parmi les enzymes mutées de séquence SEQ ID NO : 3 ou SEQ ID NO : 4, seules ou en combinaisons entre elles.

6. - Composition pharmaceutique comprenant comme principe actif au moins une enzyme PTE mutée selon la revendication 1, et possédant une activité de catalyse des composés organophosphorés, ladite au moins une enzyme mutée étant choisie parmi les enzymes mutées de séquence SEQ ID NO : 3 ou SEQ ID NO : 4, seules ou en combinaisons entre elles ; en combinaison d'un excipient pharmaceutiquement acceptable.

7. - Enzymes PTE mutées selon la revendication 1, possédant une activité de catalyse des composés organophosphorés, ladite au moins une enzyme mutée étant choisie parmi les enzymes mutées de séquence SEQ ID NO : 3 ou SEQ ID NO : 4, seules ou en combinaisons entre elles, pour son utilisation dans le traitement ou la prévention des empoisonnements par contact, inhalation ou ingestion de composés organophosphorés.

## Patentansprüche

1. Mutiertes PTE-Enzym, das von Parathionhydrolase abgeleitet ist, das die Sequenz SEQ ID NO: 1 aufweist und die folgenden Mutationen in Bezug auf die Sequenz SEQ ID NO: 1 enthält:
- Substitution von Threonin T durch Prolin P an Position 13,
- Substitution von Isoleucin I durch Valin V an Position 14,
- Substitution von Alanin A durch Serin S an Position 60,
- Substitution von Serin S durch Arginin R, an Position 79,
- Substitution von Tyrosin Y durch Histidin H an Position 124,
- Substitution von Isoleucin I durch Valin V an Position 218,
- Substitution von Histidin H durch Tyrosin Y an Position 225,
- Substitution von Glutamin Q durch Arginin R an Position 258,
- Substitution von Leucin L durch Threonin T an Position 271,
wobei das mutierte PTE-Enzym die Sequenz SED ID NO: 3 aufweist oder die folgenden Mutationen in Bezug auf die Sequenz SEQ ID NO: 1 enthält:
- Substitution von Threonin T durch Prolin P an Position 13,
- Substitution von Isoleucin I durch Valin V an Position 14,
- Substitution von Alanin A durch Serin S an Position 60,
- Substitution von Isoleucin I durch Cystein C, an Position 74,
- Substitution von Serin S durch Arginin R, an Position 79,
- Substitution von Phenylalanin F durch Valin V, an Position 100,
- Substitution von Tyrosin Y durch Histidin H an Position 124,
- Substitution von Isoleucin I durch Valin V an Position 218,
- Substitution von Histidin H durch Glutamin Q, an Position 222,
- Substitution von Histidin H durch Tyrosin Y an Position 225,
- Substitution von Alanin A durch Valin V, an Position 238,
- Substitution von Leucin L durch Methionin M, an Position 240,
- Substitution von Isoleucin I durch Asparagin N, an Position 242,
- Substitution von Glutamin Q durch Arginin R an Position 258,
- Substitution von Serin S durch Leucin L an Position 276,
wobei das mutierte PTE-Enzym die Sequenz SED ID NO: 4 aufweist.

2. Verfahren zum Erhalt eines mutierten PTE-Enzyms, umfassend das Einführen von 9 Mutationen, um die katalytische Phosphotriesterase(PTE)-Aktivität des Enzyms mit der Sequenz SEQ ID NO: 1, das in der Lage ist, phosphororganische Verbindungen zu hydrolysieren, zu erhöhen, indem in der Sequenz SEQ ID NO: 1 Folgendes substituiert wird:
- die Aminosäure an Position 13 durch Prolin P,
- die Aminosäure an Position 14 durch Valin V,
- die Aminosäure an Position 60 durch Serin S,
- die Aminosäure an Position 79 durch Arginin R,
- die Aminosäure an Position 124 durch Histidin H,
- die Aminosäure an Position 218 durch Valin V,
- die Aminosäure an Position 258 durch Arginin R,
- die Aminosäure an Position 225 durch Tyrosin Y,
- die Aminosäure an Position 271 durch Threonin T,
um ein mutiertes PTE-Enzym mit der Sequenz SEQ ID NO: 3 zu erhalten, oder umfassend das Einführen von 15 Mutationen, um die katalytische Phosphotriesterase(PTE)-Aktivität des Enzyms mit der Sequenz SEQ ID NO: 1, das in der Lage ist, phosphororganische Verbindungen zu hydrolysieren, zu erhöhen, indem in der Sequenz SEQ ID NO: 1 Folgendes substituiert wird:
- die Aminosäure an Position 13 durch Prolin P,
- die Aminosäure an Position 14 durch Valin V,
- die Aminosäure an Position 60 durch Serin S,
- die Aminosäure an Position 74 durch Cystein,
- die Aminosäure an Position 79 durch Arginin R,
- die Aminosäure an Position 100 durch Valin V,
- die Aminosäure an Position 124 durch Histidin H,
- die Aminosäure an Position 218 durch Valin V,
- die Aminosäure an Position 222 durch Glutamin Q,
- die Aminosäure an Position 225 durch Tyrosin Y,
- die Aminosäure an Position 238 durch Valin V,
- die Aminosäure an Position 240 durch Methionin M,
- die Aminosäure an Position 242 durch Asparagin N,
- die Aminosäure an Position 258 durch Asparagin R,
- die Aminosäure an Position 276 durch Leucin L,
um ein mutiertes PTE-Enzym mit der Sequenz SEQ ID NO: 4 zu erhalten, wobei die Sequenzen SEQ ID NO: 3 und SEQ ID NO: 4 eine verbesserte katalytische Aktivität bei der Hydrolyse von phosphororganischen Verbindungen im Vergleich zur katalytischen Aktivität des Enzyms mit der Sequenz SED ID NO: 1 besitzen.

3. Verwendung mindestens eines mutierten PTE-Enzyms nach Anspruch 1, das eine katalytische Phosphotriesterase(PTE)-Aktivität besitzt, die in der Lage ist, phosphororganische Verbindungen zu hydrolysieren:
- zum Dekontaminieren von Böden, die mit phosphororganischen Verbindungen verunreinigt sind,
- zum Dekontaminieren einer Oberfläche, von Haut, von Schleimhäuten oder Haaren, die mit phosphororganischen Verbindungen kontaminiert ist/sind, oder
- zum Vorbeugen oder Behandeln einer inneren oder äußeren Vergiftung durch Einnahme oder Einatmen einer phosphororganischen Verbindung, oder
- zum Kontrollieren der Verunreinigung von Wasser, das mit phosphororganischen Verbindungen verunreinigt ist, oder
- zum Vernichten von Beständen an Nervengiften,
wobei das mindestens eine mutierte PTE-Enzym ausgewählt ist aus den mutierten Enzymen mit der Sequenz SEQ ID NO: 3 oder SEQ ID NO: 4, allein oder in Kombinationen miteinander.

4. Kit zum Dekontaminieren von Oberflächen, von Haut oder Schleimhäuten, Haaren, die mit phosphororganischen Verbindungen kontaminiert sind, von Geweben, Filtern oder Farben, wobei das Kit mindestens ein mutiertes PTE-Enzym nach Anspruch 1 umfasst, das eine Aktivität zur Katalyse von phosphororganischen Verbindungen aufweist und wobei das mindestens eine mutierte Enzym ausgewählt ist aus den mutierten Enzymen mit der Sequenz SEQ ID NO: 3 oder SEQ ID NO: 4, allein oder in Kombinationen miteinander.

5. Pflanzenschutzmittel, das als Wirkstoff mindestens ein mutiertes PTE-Enzym nach Anspruch 1 umfasst, und eine Aktivität zur Katalyse von phosphororganischen Verbindungen besitzt, wobei das mindestens eine mutierte Enzym ausgewählt ist aus den mutierten Enzymen mit der Sequenz SEQ ID NO: 3 oder SEQ ID NO: 4, allein oder in Kombinationen miteinander.

6. Pharmazeutische Zusammensetzung, die als Wirkstoff mindestens ein mutiertes PTE-Enzym nach Anspruch 1 umfasst, und eine Aktivität zur Katalyse von phosphororganischen Verbindungen besitzt, wobei das mindestens eine mutierte Enzym ausgewählt ist aus den mutierten Enzymen mit der Sequenz SEQ ID NO: 3 oder SEQ ID NO: 4, allein oder in Kombinationen miteinander; in Kombination mit einem pharmazeutisch annehmbaren Hilfsstoff.

7. Mutierte PTE-Enzyme nach Anspruch 1, die eine Aktivität zur Katalyse von phosphororganischen Verbindungen besitzen, wobei das mindestens eine mutierte Enzym ausgewählt ist aus den mutierten Enzymen mit der Sequenz SEQ ID NO: 3 oder SEQ ID NO: 4, allein oder in Kombinationen miteinander, zur Verwendung bei der Behandlung oder Vorbeugung von Vergiftungen durch Kontakt, Inhalation oder Ingestion von phosphororganischen Verbindungen.

## Claims

1. Mutated PTE enzyme derived from parathion hydrolase having sequence SEQ ID NO: 1, and containing the following mutations with respect to sequence SEQ ID NO: 1:
- Substitution of threonine T by proline P, in position 13,
- Substitution of isoleucine I by valine V, in position 14,
- Substitution of alanine A by serine S, in position 60,
- Substitution of serine S by arginine R, in position 79,
- Substitution of tyrosine Y by histidine H, in position 124,
- Substitution of isoleucine I by valine V, in position 218,
- Substitution of histidine H by tyrosine Y, in position 225,
- Substitution of glutamine Q by arginine R, in position 258,
- Substitution of leucine L by threonine T, in position 271,
said mutated PTE enzyme having sequence SEQ ID NO: 3,
or containing the following mutations with respect to sequence SEQ ID NO: 1:
- Substitution of threonine T by proline P, in position 13,
- Substitution of isoleucine I by valine V, in position 14,
- Substitution of alanine A by serine S, in position 60,
- Substitution of isoleucine I by cysteine C in position 74
- Substitution of serine S by arginine R, in position 79,
- Substitution of phenylalanine F by valine V, in position 100,
- Substitution of tyrosine Y by histidine H, in position 124,
- Substitution of isoleucine I by valine V, in position 218,
- Substitution of histidine H by glutamine Q, in position 222,
- Substitution of histidine H by tyrosine Y, in position 225,
- Substitution of alanine A by valine V, in position 238,
- Substitution of leucine L by methionine M, in position 240,
- Substitution of isoleucine I by asparagine N, in position 242,
- Substitution of glutamine Q by arginine R, in position 258,
- Substitution of serine S by leucine L, in position 276,
said mutated PTE enzyme having sequence SEQ ID NO: 4.

2. Process for obtaining a mutated PTE enzyme comprising the introduction of 9 mutations to increase the phosphotriesterase (PTE) catalytic activity of the enzyme of sequence SEQ ID NO: 1 capable of hydrolyzing organophosphorus compounds, by substituting in sequence SEQ ID NO: 1:
- the amino acid in position 13 by proline P,
- the amino acid in position 14 by valine V,
- the amino acid in position 60 by serine S,
- the amino acid in position 79 by arginine R,
- the amino acid in position 124 by histidine H,
- the amino acid in position 218 by valine V,
- the amino acid in position 258 by arginine R,
- the amino acid in position 225 by tyrosine Y,
- the amino acid in position 271 by threonine T,
to obtain a mutated PTE enzyme of sequence SEQ ID NO: 3,
or comprising the introduction of 15 mutations to increase the phosphotriesterase (PTE) catalytic activity of the enzyme of sequence SEQ ID NO: 1 capable of hydrolyzing organophosphorus compounds, by substituting in sequence SEQ ID NO: 1 :
- the amino acid in position 13 by proline P,
- the amino acid in position 14 by valine V,
- the amino acid in position 60 by serine S,
- the amino acid in position 74 by cysteine,
- the amino acid in position 79 by arginine R,
- the amino acid in position 100 by valine V,
- the amino acid in position 124 by histidine H,
- the amino acid in position 218 by valine V,
- the amino acid in position 222 by glutamine Q,
- the amino acid in position 225 by tyrosine Y,
- the amino acid in position 238 by valine V,
- the amino acid in position 240 by methionine M,
- the amino acid in position 242 by asparagine N,
- the amino acid in position 258 by arginine R,
- the amino acid in position 276 by leucine L,
to obtain a mutated PTE enzyme of sequence SEQ ID NO: 4,
said sequence SEQ ID NO: 3 and SEQ ID NO: 4 have an improved hydrolysis catalytic activity of organophosphorus compounds, compared to the catalytic activity of the enzyme of sequence SEQ ID NO: 1.

3. Use of at least one mutated PTE enzyme according to claim 1, and having a phosphotriesterase (PTE) catalytic activity capable of hydrolyzing organophosphorus compounds :
- for the decontamination of soils polluted with organophosphorus compounds, or
- for the decontamination of a surface, skin, mucous membranes or hair contaminated with organophosphorus compounds, or for the prevention or the treatment of an internal or of an external poisoning by ingestion or inhalation of an organophosphorus compound, or
- for the control of pollution of water polluted with organophosphorus compounds, or
- for the destruction of stocks of neurotoxic agents,
said at least one mutated PTE enzyme being chosen among mutated enzyme of sequence SEQ ID NO: 3 or SEQ ID NO: 4, alone or in combination thereof.

4. Kit for the decontamination of surfaces, skin or mucous membranes contaminated with organophosphorus compounds, said kit comprising at least one mutated PTE enzyme according to claim 1, having a catalysis activity of organophosphorus compounds and said at least one mutated enzyme being chosen among the mutated enzymes of sequence SEQ ID NO: 3 or SEQ ID NO: 4, alone or in combination thereof.

5. Phytosanitary composition comprising as active ingredient at least one mutated PTE enzyme according to claim1, and having a catalysis activity of organophosphorus compounds, said at least one mutated enzyme being chosen among the mutated enzymes of sequence SEQ ID NO: 3 or SEQ ID NO: 4, alone or in combination thereof.

6. Pharmaceutical composition comprising as active ingredient at least one mutated PTE enzyme according to claim1, and having a catalysis activity of organophosphorus compounds, said at least one mutated enzyme being chosen among the mutated enzymes of sequence SEQ ID NO: 3 or SEQ ID NO: 4, alone or in combination thereof, in combination with a pharmaceutically acceptable excipient.

7. Mutated PTE enzyme according to claim1, having a catalysis activity of organophosphorus compounds, said at least one mutated enzyme being chosen among the mutated enzymes of sequence SEQ ID NO: 3 or SEQ ID NO: 4, alone or in combination thereof, for its use in the treatment or the prevention of poisonings by contact, inhalation or ingestion of organophosphorus compounds.
